# EUROPEAN PATENT APPLICATION

(11) **EP 2 574 359 A1**
(43) Date of publication of application: **03.04.2013**
(21) Application number: 11182977.6
(22) Date of filing: 27.09.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a needle safety device (2) comprising a needle hub (2.2), a needle (2.3) coupled to the needle hub (2.2) and having a distal tip, a sleeve (2.9) coupled to the needle hub (2.2), a needle shield (2.1) telescopically coupled to the sleeve (2.9); and a needle disabling mechanism coupled to the needle (2.3). In a first axial position (PA1), the needle shield (2.1) covers the distal tip of the needle (2.3) and the needle disabling mechanism is in a first angular position. In a retracted position (PR), the needle shield (2.1) is retracted relative to the needle (2.3) exposing the distal tip of the needle (2.3). In a second axial position (PA2), the needle shield (2.1) is distal of the first axial position (PA1) and the needle disabling mechanism is in a second angular position bending the needle (2.3).

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal safety needle assembly.

### Summary of the Invention

It is an object of the present invention to provide an improved safety needle assembly that minimizes the risk of an accidental needle stick injury, that is safe to handle, and that provides needle safety before and after the medicament is delivered.

In an exemplary embodiment, a needle safety device according to the present invention comprises a needle hub, a needle coupled to the needle hub and having a distal tip, a sleeve coupled to the needle hub, a needle shield telescopically coupled to the sleeve, and a needle disabling mechanism coupled to the needle. In a first axial position, the needle shield covers the distal tip of the needle and the needle disabling mechanism is in a first angular position. In a retracted position, the needle shield is retracted relative to the needle exposing the distal tip of the needle. In a second axial position, the needle shield is distal of the first axial position and the needle disabling mechanism is in a second angular position bending the needle. In an exemplary embodiment, the needle hub is adapted to engage a medicament delivery device.

In an exemplary embodiment, the sleeve includes one or more resilient latch elements. In the first axial position, the needle disabling mechanism abuts the latch elements into a deflected position.

In an exemplary embodiment, the needle shield includes a first guide slot and a second guide slot. The needle disabling mechanism includes a holder, a needle disabler rotatably coupled to the holder, and a second spring element adapted to bias the needle disabler in the first angular position relative to the holder. The needle disabler has a channel adapted to engage the needle. The first guide slot is adapted to engage the holder. The second guide slot is adapted to engage the needle disabler. The second guide slot includes a distal section having a first width and a proximal section having a second width, and the second width is greater than the first width. The needle disabler has a width substantially equal to the first width, and in the first axial position and the retracted position, the needle disabler engages the distal section. When moving from the retracted position to the second axial position, the holder abuts the latch preventing distal movement of the needle disabling mechanism relative to the needle and causing the needle disabler to move into the proximal section of the second guide slot. In the second axial position, the needle disabler is in the proximal section and rotates under a force of the second spring element into the second angular position.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a semitransparent perspective view of an exemplary embodiment of a needle safety device before use,
- Figure 2: shows a semitransparent perspective view of an exemplary embodiment of a needle safety device before use during pushing against an injection site,
- Figure 3: shows a semitransparent perspective view of an exemplary embodiment of a needle safety device during use,
- Figure 4: shows a semitransparent perspective view of an exemplary embodiment of a needle safety device after use,
- Figure 5: shows a semitransparent perspective view of an exemplary embodiment of a needle safety device after use in a needle disabling procedure,
- Figure 6: shows a semitransparent perspective view of an exemplary embodiment of a needle safety device after a needle disabling procedure,
- Figure 7: shows a semitransparent perspective view of an exemplary embodiment of a needle safety device after a needle disabling procedure, and
- Figure 8: shows a semitransparent perspective view of an exemplary embodiment of a needle safety device after a needle disabling procedure.

Corresponding parts are marked with the same reference symbols in all figures.

### Detailed Description

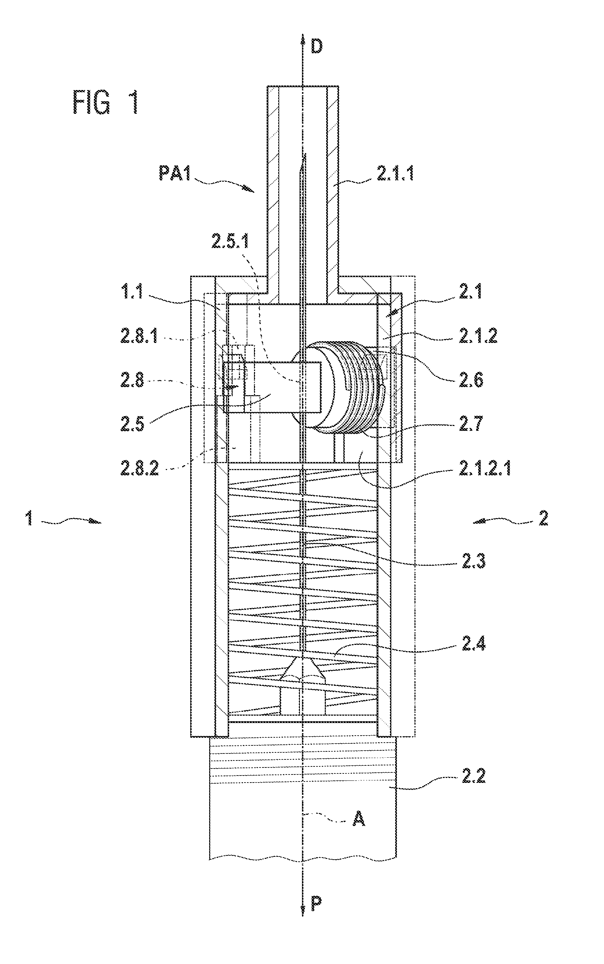

Figure 1 shows a semitransparent perspective view of an exemplary embodiment of a medicament delivery device 1. The delivery device 1 comprises a housing 1.1 and may be arranged as a pen injector, a syringe, a dental syringe, an auto-injector or a similar device suitable for delivering a medicament to a patient.

An exemplary embodiment of a needle safety device 2 is coupled to the delivery device 1. The safety device 2 has a longitudinal axis A extending from a proximal direction P to a distal direction D. In an exemplary embodiment, the safety device 2 comprises a needle shield 2.1 telescopically arranged in a sleeve 2.9 mounted to a needle hub 2.2. A needle 2.3 is mounted to the needle hub 2.2, and the needle hub 2.2 is adapted to engage the delivery device 1 (e.g., via threads, snap-fit, bayonet coupling, friction, etc.). In another exemplary embodiment, the needle hub 2.2 may be intergrally formed with the housing 1.1 of the delivery device 1. The needle 2.3 may be aligned on the axis A.

In an exemplary embodiment, the needle shield 2.1 includes two sections 2.1.1, 2.1.2 with different diameters, wherein the diameter of the distal section 2.1.1 is smaller than the diameter of the proximal section 2.1.2. An open distal end of the distal section 2.1.1 may be concentrically aligned with respect to the axis A so that the needle 2.3 is allowed to pass through the open distal end of the needle shield 2.1 during an injection procedure.

In an exemplary embodiment, the needle shield 2.1 may be contained in and axially translatable relative to the sleeve 2.9. The sleeve 2.9 may be fixed to or integral with the needle hub 2.2. The sleeve 2.9 includes an aperture 2.9.1 formed in a distal end of the sleeve 2.9 which is adapted to receive the distal section 2.1.1 of the needle shield 2.1.

In an exemplary embodiment, the needle shield 2.1 is biased with respect to the needle hub 2.2 by a first spring element 2.4. The first spring element 2.4 is arranged as a compression spring and bears against a proximal end of the proximal section 2.1.2 of the needle shield 2.1 in the distal direction D and against the needle hub 2.2 in the proximal direction P. Prior to use, the first spring element 2.4 may be arranged in an unstressed state or a pre-loaded state.

Figure 1 shows the safety device 1 in a first axial position (PA1) in which the needle shield 1 is maintained in a distal portion of the sleeve 2.9 by abutting the distal end of the sleeve 2.9 and the first spring element 2.4. As explained below, during an injection procedure, the first spring element 2.4 is compressed and energized when the needle shield 2.1 is pressed against an injection site, causing the needle shield 2.1 to move proximally relative to the sleeve 2.9.

The safety device 2 includes a needle disabling mechanism which is adapted to render the needle 2.3 inoperative after use of the safety device 2, thereby preventing inadvertent exposure of the needle 2.3 and needlestick injuries.

In an exemplary embodiment, the needle disabling mechanism comprises a needle disabler 2.5 coupled to a holder 2.6 and rotationally biased with respect to the holder 2.6 by a second spring element 2.7. The second spring element 2.7, e.g., a torsion spring, may be arranged in a pre-stressed state in a first angular position before actuation of the disabling mechanism.

As shown in the first axial position (PA1) in Figure 1, in an exemplary embodiment, the holder 2.6 may be received in a first guide slot 2.1.2.1 formed in the needle shield 2.1. The first guide slot 2.1.2.1 may include a distal abutment face and an open proximal end, thus preventing movement of the holder 2.6 relative to the needle shield 2.1 in the distal direction D and allowing movement of the holder 2.6 relative to the needle shield 2.1 in the proximal direction P.

The needle disabler 2.5 includes a channel 2.5.1 which engages the needle 2.3. The channel 2.5.1 may be formed to slightly, frictionally engage the needle 2.3 to prevent movement of the needle disabling mechanism relative to the needle 2.3 prior to use.

A lateral end of the disabler 2.5 opposite the holder 2.6 is adapted to engage a second guide slot 2.8 formed in the proximal section 2.1.2 of the needle shield 2.1. The second guide slot 2.8 includes a distal section 2.8.1 having a first width and a proximal section 2.8.2 having a second width, and the first width is less than the second width. Also, a width of the needle disabler 2.5 is substantially equal to the first width. In the first axial position (PA1), the needle disabler 2.5 is in a first angular position, because it is engaged in the distal section 2.8.1 and thus prevented from rotating relative to the holder 2.6.

One or more resilient latch elements 2.9.2 may be formed on an inner surface of the sleeve 2.9. In the first axial position (PA1), the holder 2.6 abuts the latch element 2.9.2, deflecting the latch element 2.9.2 radially away from the axis A.

In an exemplary embodiment, the needle shield 2.1 includes ribs (not shown) which engage channels (not shown) formed on the inner surface of the sleeve 2.9 to prevent the needle shield 2.1 from rotating relative to the sleeve 2.9.

As shown in Figure 1, in the first axial position (PA1), a distal tip of the needle 2.3 is covered by the distal section 2.1.1 of the needle shield 2.1.

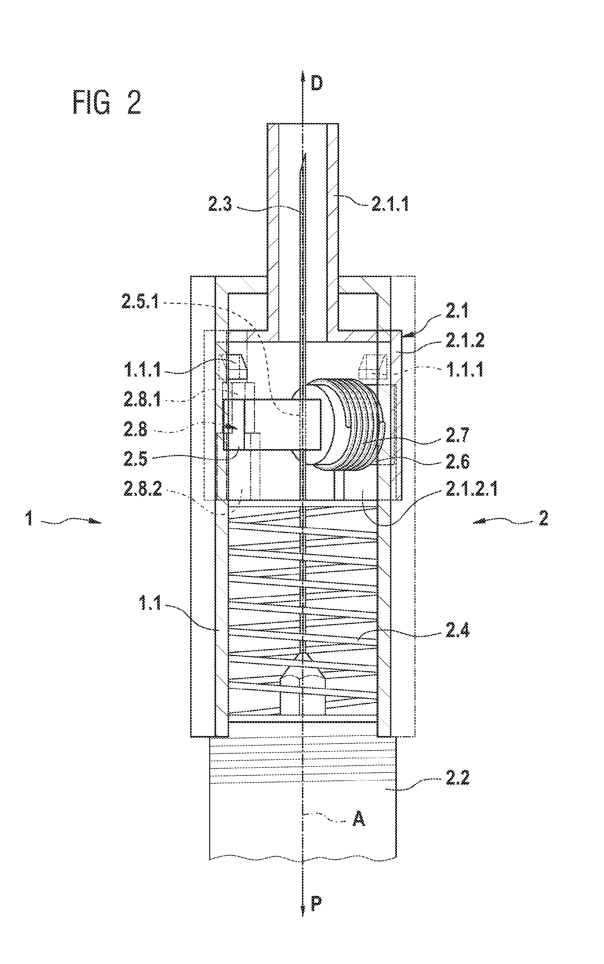

Figure 2 shows the safety device 2 when the needle shield 2.1 is pushed against an injection site. When axial force is applied to the needle shield 2.1 toward the proximal direction P, the needle shield 2.1 moves proximally relative to the sleeve 2.9, compressing the first spring element 2.4. Also, the needle disabler 2.5 abuts the distal section 2.8.1 of the second guide slot 2.8 and the holder 2.6 abuts a distal end of the first guide slot 2.1.2.1, causing the needle disabling mechanism to move proximally relative to the needle 2.3. The holder 2.6 disengages the latch element 2.9.2, allowing the latch element 2.9.2 to return to its non-deflected position.

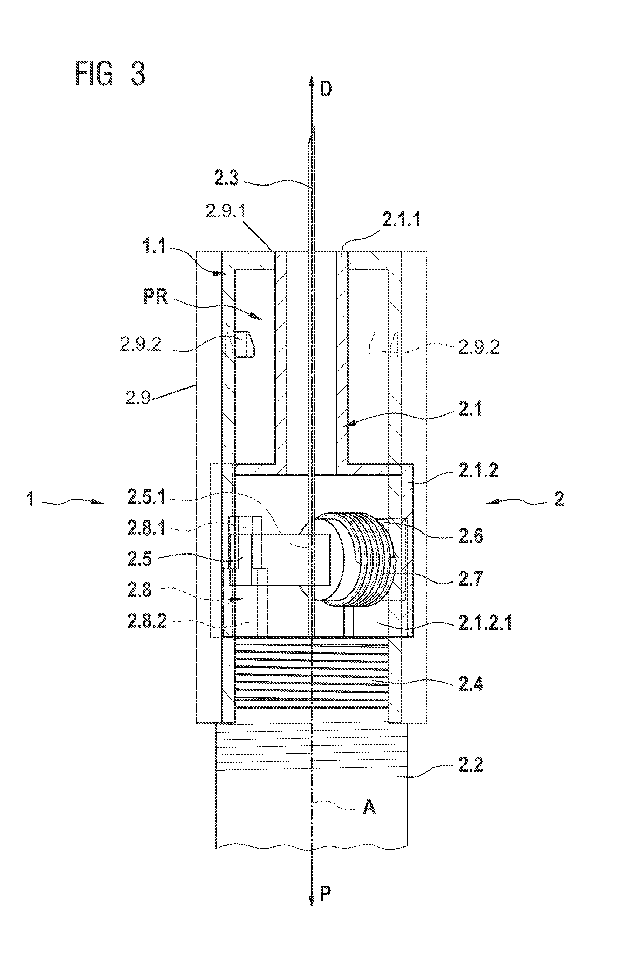

Figure 3 shows the safety device 2 in a retracted position (PR) relative to the sleeve 2.9 and in which the distal tip of the needle 2.3 is exposed beyond the sleeve 2.9 and the distal section 2.1.1 of the needle shield 2.1. The first spring element 2.4 is compressed and energized during the proximal movement of the needle shield 2.1 with respect to the sleeve 2.9. The needle disabler 2.5 remains engaged in the distal section 2.8.1 of the second guide slot 2.8, and the holder 2.6 remains engaged in the first guide slot 2.1.2.1.

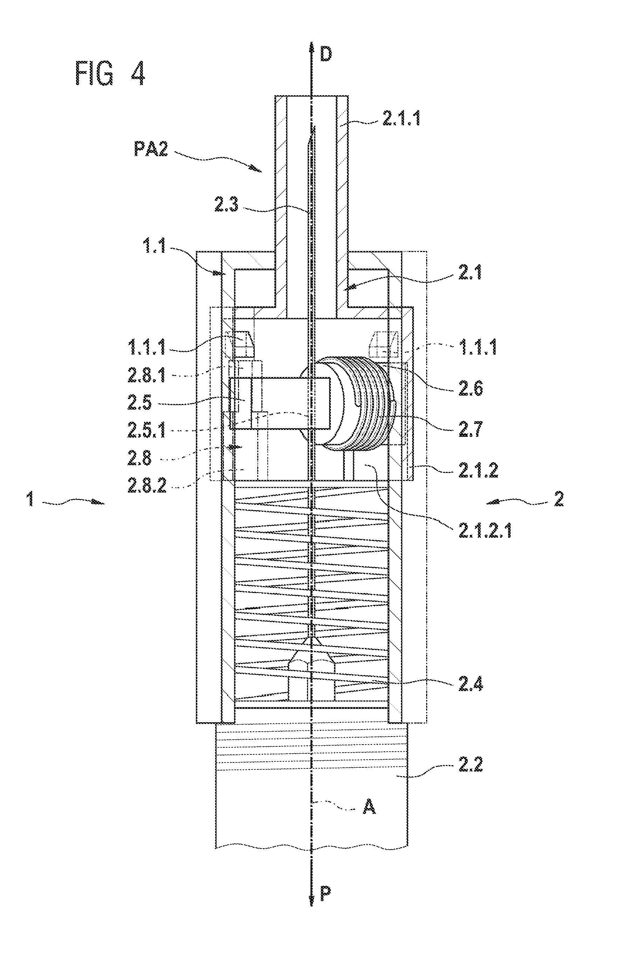

Figure 4 shows safety device 2 when it is being removed from the injection site. Under the force of the first spring element 2.4, the needle shield 2.1 moves distally relative to the sleeve 2.9. The needle disabling mechanism moves distally relative to the needle 2.3.

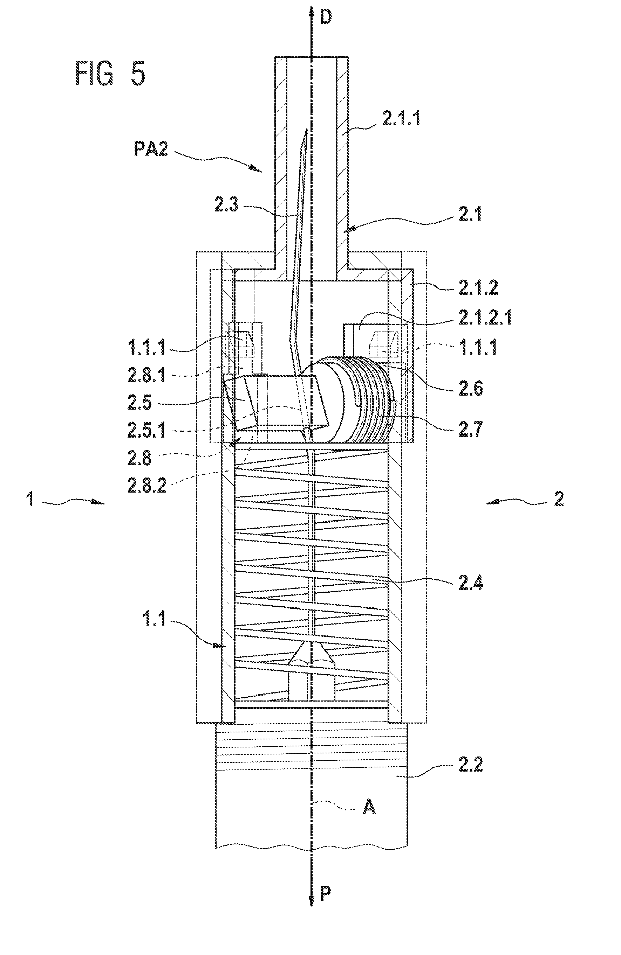

Figure 5 shows the safety device 2 in a second axial position (PA2). As the needle shield 2.1 moves distally relative to the sleeve 2.9 and the needle disabling mechanism moves distally relative to the needle 2.3, the holder 2.6 abuts the latch element 2.9.2, preventing further movement of the needle disabling mechanism relative to the needle 2.3. However, the needle shield 2.1 continues moving distally relative to the sleeve 2.9 under the force of the first spring element 2.4. Thus, as the needle shield 2.1 moves distally relative to the needle disabling mechanism, the needle disabler 2.5 moves into the proximal section 2.8.2 of the second guide slot 2.8. Because the proximal section 2.8.2 is wider than the distal section 2.8.1, the needle disabler 2.5 rotates into a second angular position under the force of the second spring element 2.7. Rotation of the needle disabler 2.5 bends the needle 2.3, pulling the distal tip of the needle 2.3 proximally into the needle shield 2.1.

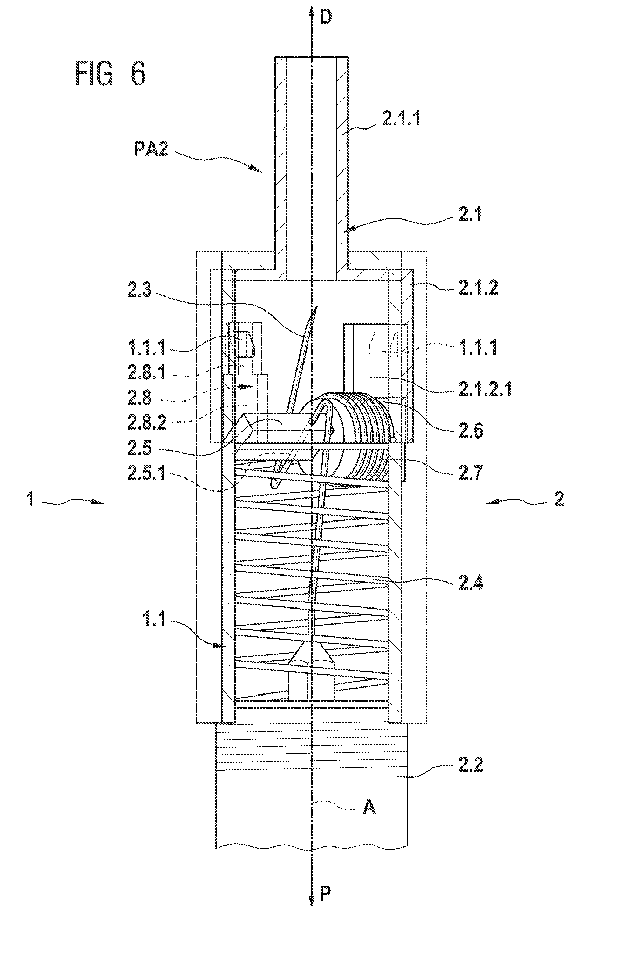

Figure 6 shows the safety device 2 after the needle disabler 2.5 has bent the needle 2.3. During bending, the distal tip of the needle 2.3 may be pulled into the proximal section 2.1.2 of the needle shield 2.1.

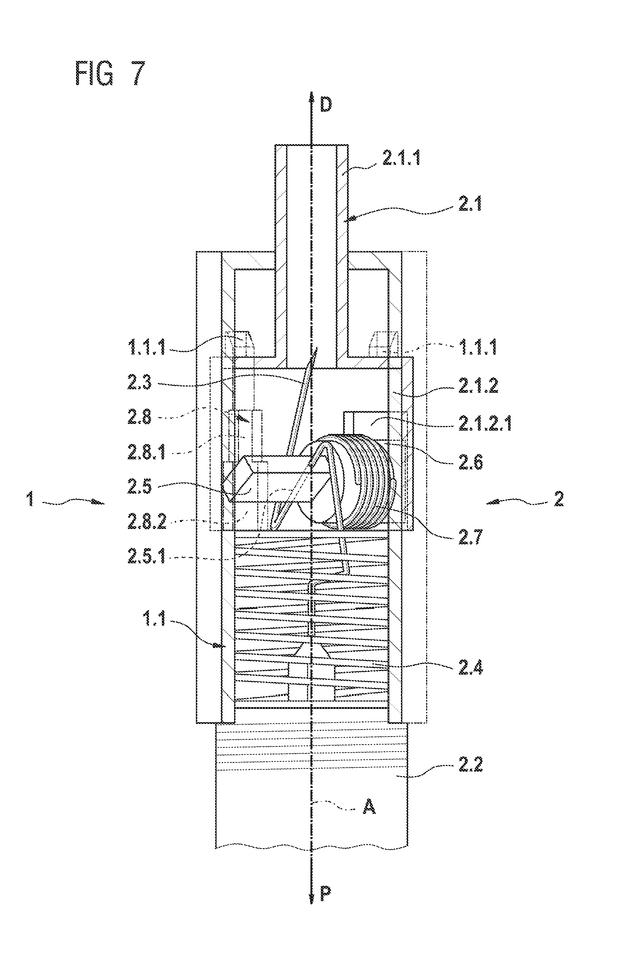

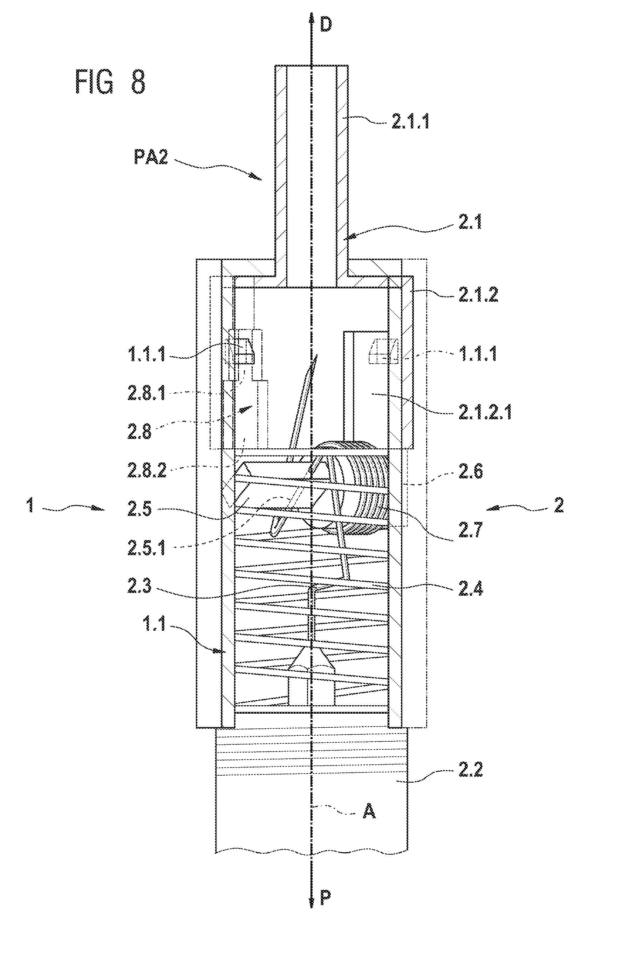

Figures 7 and 8 show the safety device 2 if a subsequent axial force is applied on the needle shield 2.1 when attempting to re-expose the needle 2.3. The needle 2.3 is bent one more time, because the lateral end of the needle disabler 2.5 cannot slide into the distal section 2.8.1 again when the needle shield 2.1 moves axially in the proximal direction P. The distal section 2.8.1 abuts the rotated needle disabler 2.5, and the torque from the second spring element 2.7 causes rotation of the needle disabler 2.5 to further bend the needle 2.3.

Thus, the safety device 2 improves needle safety and in particular minimizes the risk that a used needle 2.3 is exposed, so that a transmission of blood-borne diseases, like for example HIV, AIDS, Hepatitis B or Hepatitis C may be avoided.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle safety device (2) comprising:
a needle hub (2.2);
a needle (2.3) coupled to the needle hub (2.2), the needle (2.3) having a distal tip;
a sleeve (2.9) coupled to the needle hub (2.2);
a needle shield (2.1) telescopically coupled to the sleeve (2.9); and
a needle disabling mechanism coupled to the needle (2.3),
wherein, in a first axial position (PA1), the needle shield (2.1) covers the distal tip of the needle (2.3) and the needle disabling mechanism is in a first angular position;
wherein, in a retracted position (PR), the needle shield (2.1) is retracted relative to the needle (2.3) exposing the distal tip of the needle (2.3);
wherein, in a second axial position (PA2), the needle shield (2.1) is distal of the first axial position (PA1) and the needle disabling mechanism is in a second angular position bending the needle (2.3).

2. The needle safety device (2) according to claim 1, wherein the needle hub (2.2) is adapted to engage a medicament delivery device.

3. The needle safety device (2) according to any one of the preceding claims, wherein the sleeve (2.9) includes one or more resilient latch elements (2.9.2).

4. The needle safety device (2) according to claim 3, wherein, in the first axial position (PA1), the needle disabling mechanism abuts the latch elements (2.9.2) into a deflected position.

5. The needle safety device (2) according to any one of the preceding claims, wherein the needle shield (2.1) includes a first guide slot (2.1.2.1) and a second guide slot (2.8).

6. The needle safety device (2) according to any one of the preceding claims, wherein the needle disabling mechanism includes:
a holder (2.6);
a needle disabler (2.5) rotatably coupled to the holder (2.6), the needle disabler (2.5) having a channel (2.5.1) adapted to engage the needle (2.3); and
a second spring element (2.7) adapted to bias the needle disabler (2.5) in the first angular position relative to the holder (2.6).

7. The needle safety device (2) according to claims 5 and 6, wherein the first guide slot (2.1.2.1) is adapted to engage the holder (2.6).

8. The needle safety device (2) according to claims 5 and 6, wherein the second guide slot (2.8) is adapted to engage the needle disabler (2.5), the second guide slot (2.8) including a distal section (2.8.1) having a first width and a proximal section (2.8.2) having a second width, and the second width is greater than the first width.

9. The needle safety device (2) according to claims 8, wherein the needle disabler (2.5) has a width substantially equal to the first width, and in the first axial position (PA1) and the retracted position (PR), the needle disabler (2.5) engages the distal section (2.8.1).

10. The needle safety device (2) according to claims 3 and 8, wherein, when moving from the retracted position (PR) to the second axial position (PA2), the holder (2.6) abuts the latch (2.9.2) preventing distal movement of the needle disabling mechanism relative to the needle (2.3) and causing the needle disabler (2.5) to move into the proximal section (2.8.2) of the second guide slot (2.8).
The needle safety device (2) according to claim 10, wherein, in the second axial position (PA2), the needle disabler (2.5) is in the proximal section (2.8.2) and rotates under a force of the second spring element (2.7) into the second angular position.
